# EUROPEAN PATENT APPLICATION

(11) **EP 4 733 398 A1**
(43) Date of publication of application: **29.04.2026**
(21) Application number: 24831845.3
(22) Date of filing: 21.06.2024
(51) Int. Cl.: C12N 11/04, C12N 5/071, C12N 11/10

(54) **METHOD FOR PRODUCING CELL CAPSULE, METHOD FOR PRODUCING CELL POPULATION OR CELL PRODUCT, SOLUTION, AND CELL POPULATION**

(30) Priority: 26.06.2023 JP 2023104592
(71) Applicant: Cellfiber Co., Ltd., Tokyo 135-0031 (JP)
(72) Inventor: IKEDA Kazuhiro, Tokyo 135-0031 (JP); IZUMI Nozomi, Tokyo 135-0031 (JP)
(74) Representative: Isarpatent
(86) International application number: PCT/JP2024/022522
(87) International publication number: WO 2025/004984

(57) **Abstract**

Provided is a method for producing a cell capsule capable of alleviating an adverse effect on a cell. This method for producing a cell capsule comprises continuously or intermittently discharging a hydrogel or a hydrogel precursor covering a cell into a collection solution. The collection solution contains a pH buffer.

## Description

### Technical Field

The present invention relates to a method for producing a cell capsule, a method for producing a cell population or a cell product, a solution used in the method for producing a cell capsule, and a cell population.

### Background Art

At present, in various fields such as developmental biology, drug discovery, and regenerative medicine, attempts are being made to culture various types of cells. These cells are typically cultured two-dimensionally on the surface of a tissue culture plastic container. A technique is known in which cells are cultured under a three-dimensional or pseudo-three-dimensional culture environment using a scaffold such as a porous membrane or a hydrogel. Patent Literature 1 to Patent Literature 6 below disclose culturing cells inside tubular or spherical shell-like hydrogels.

In recent years, instead of monolayer culture in which cells are cultured two-dimensionally, spheroid culture, in which cells are cultured and aggregated three-dimensionally, has attracted attention. Spheroid culture, compared to monolayer culture, is known to be capable of constructing a state closer to that of cells in vivo and of eliciting specific functions that cells exhibit in vivo. Therefore, for example, in drug discovery research and the like, cell aggregates are expected as useful tools.

### Citation List

### Patent Literature

Patent Literature 1: WO 2011/046105 A1
Patent Literature 2: WO 2017/091662 A1
Patent Literature 3: WO 2018/098295 A1
Patent Literature 4: WO 2019/178549 A1
Patent Literature 5: WO 2020/032221 A1
Patent Literature 6: WO 2009/108138 A1
Patent Literature 7: JP 2017-99303 A

### Summary

The inventors of the present application have found the following new problems regarding the production of tubular or spherical shell-like hydrogels as described in Patent Literature 1 to Patent Literature 6. In Patent Literature 1 to Patent Literature 6, a hydrogel precursor is discharged continuously or intermittently from a nozzle into a solution, and a tubular or spherical shell-like hydrogel is produced within the solution. When the hydrogel precursor is discharged for a long period of time for the purpose of encapsulating a large number of cells within the hydrogel, for example, for the purpose of mass culturing of cells, the pH (hydrogen ion index) in the solution fluctuates, which can adversely affect the cells encapsulated in the hydrogel.

Therefore, there is a demand for methods for producing cell capsules, methods for producing cell populations or cell products, and the like, which can mitigate adverse effects on cells.

A method for producing a cell capsule according to one aspect includes continuously or intermittently discharging a hydrogel or a hydrogel precursor covering cells into a collection solution, in which the collection solution contains a pH buffer.

A method for producing a cell population or a cell product according to one aspect includes culturing a cell inside a hydrogel using a cell capsule produced by the method for producing a cell capsule described above.

A cell population according to one aspect may be a cell population produced by the method for producing a cell population or a cell product.

A solution according to one aspect is a solution used for producing a cell capsule having a hydrogel covering a cell, and contains a pH buffer.

### Brief Description of Drawings

Fig. 1 is a schematic view showing a configuration of a cell capsule according to one aspect.
Fig. 2 is a schematic cross-sectional view of the cell capsule shown in Fig. 1.
Fig. 3 is a schematic view showing a configuration of a cell capsule according to another aspect.
Fig. 4 is a schematic view for describing a method for producing a cell capsule according to a first embodiment.
Fig. 5 is a schematic view for describing a method for producing a cell capsule according to a second embodiment.
Fig. 6 is a schematic view for describing a method for producing a cell capsule according to a third embodiment.
Fig. 7 is a graph showing measurement results of a lactate amount in a culture medium of human iPS cells on Day 7 in Reference Examples 1 to 6 and Examples 1 to 6.
Fig. 8 is a graph showing measurement results of a proliferation rate of human iPS cells on Day 7 in Reference Examples 1 to 6 and Examples 1 to 6.
Fig. 9 is a graph showing measurement results of the lactate amount in the culture medium of human iPS cells on Day 7 in Examples 7 to 10.
Fig. 10 is a graph showing measurement results of the lactate amount in the culture medium of human iPS cells on Day 7 in Reference Examples 7 to 10.
Fig. 11 is an enlarged photograph showing cell capsules on Day 2 in Examples 11 to 22.
Fig. 12 is a graph showing measurement results of the lactate amount in the culture medium of K562 cells on Day 2 in Examples 11 to 22.
Fig. 13 is a graph showing the measurement results of the lactate amount in the culture medium of K562 cells on Day 2 in Reference Examples 11 to 22.

### Description of Embodiments

Hereinafter, embodiments will be described with reference to the drawings. In the following drawings, the same or similar parts are denoted by the same or similar reference numerals. However, it should be noted that the drawings are schematic, and ratios of dimensions and the like may be different from actual ones.

The cell capsule has cells covered with a hydrogel. One or more cells may be covered with a hydrogel. The cells may be dispersed cells or may be in the form of cell aggregates. The hydrogel may cover cells, and may have, for example, a substantially tubular shape, a substantially string-like shape, a substantially spherical upper shape, or a substantially spherical shape.

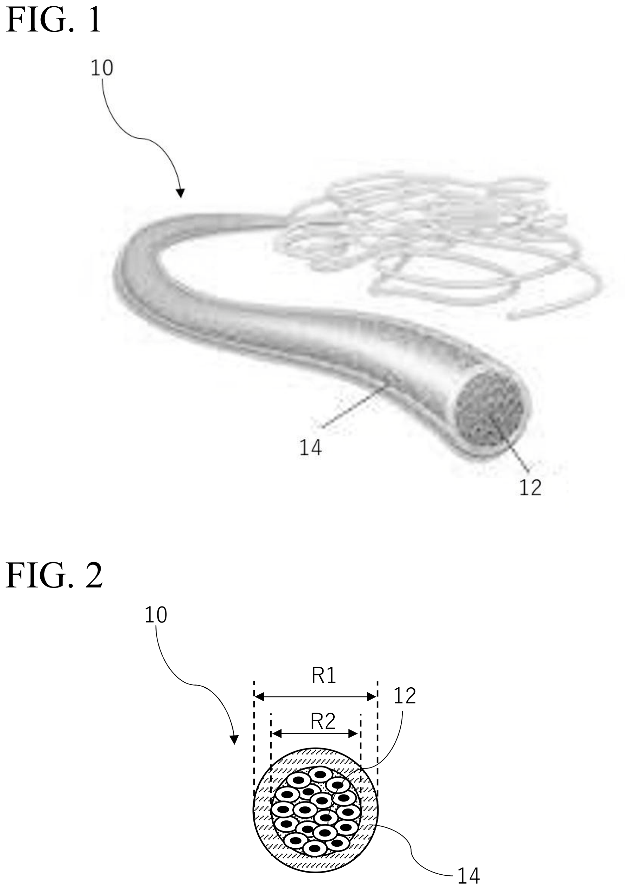

Fig. 1 is a schematic view showing a configuration of a cell capsule according to one aspect. Fig. 2 is a schematic cross-sectional view of the cell capsule shown in Fig. 1. In the aspects shown in Figs. 1 and 2, a hydrogel 14 has a substantially tubular shape. Specifically, the cell capsule may have the tubular hydrogel 14 and a core 12 covered with the hydrogel 14. The cells are provided in the core 12. The hydrogel 14 surrounds the periphery of the core 12. More specifically, the hydrogel 14 surrounds the core 12 in a cross section orthogonal to the direction in which the hydrogel 14 extends. The tubular hydrogel 14 extends longitudinally, and the core 12 extends longitudinally in a string shape inside the tubular hydrogel 14. One or more cells (including spheroids) may be contained in one cell capsule.

The length of the hydrogel 14 may be, for example, 5 cm or more, more preferably 10 cm or more, and still more preferably 20 cm or more. The longer the length of the hydrogel, the more cells can be contained inside one hydrogel. The length of the hydrogel 14 is not particularly limited, but may be, for example, 10 m or less.

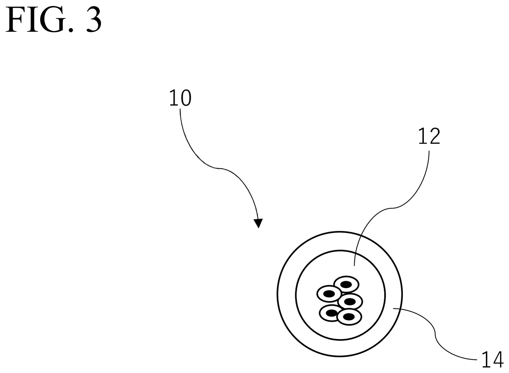

Fig. 3 is a schematic view showing a configuration of a cell capsule according to another aspect. In the aspect shown in Fig. 3, the cell capsule has a substantially spherical shape. The hydrogel 14 has a substantially spherical shell-like shape. Specifically, the cell capsule may have the spherical shell-like hydrogel 14 and the core 12 covered with the hydrogel 14. The core 12 has a substantially spherical shape and is provided inside the spherical shell-like hydrogel 14. The cells are provided in the core 12. The hydrogel 14 surrounds the entire core 12. It is also conceivable that the cell capsule according to the aspect shown in Fig. 3 be obtained by shortening the length of the cell capsule according to the aspect shown in Figs. 1 and 2. One or more cells (including spheroids) may be contained in one cell capsule.

The outer diameter of the hydrogel 14 is not particularly limited, but may be, for example, in a range of 40 µm to 4,000 µm, preferably in a range of 80 µm to 2,000 µm, and more preferably in a range of 100 µm to 1,000 µm. When the hydrogel has a tubular shape, the outer diameter of the hydrogel may be defined by the outer diameter of the hydrogel in a cross section orthogonal to the direction in which the cell capsule extends (refer to reference numeral R1 in Fig. 2).

The inner diameter of the hydrogel is not particularly limited, but may be, for example, in the range of 10 µm to 3,000 µm, preferably in the range of 20 µm to 1,000 µm, and more preferably in the range of 40 µm to 500 µm. When the hydrogel has a tubular shape, the inner diameter of the hydrogel may be defined by the inner diameter of the hydrogel in a cross section orthogonal to the direction in which the cell capsule extends (refer to reference numeral R2 in Fig. 2). The inner diameter and the outer diameter described above can be measured by, for example, a phase contrast optical microscope.

The hydrogel 14 is obtained by gelating a hydrogel precursor. The hydrogel 14 preferably has sufficient permeability to cell culture medium components.

The hydrogel 14 may contain at least one selected from the group consisting of alginate gel, Matrigel, collagen gel, chitosan gel, gelatin, peptide gel, laminin gel, agarose gel, nanocellulose, methylcellulose, gellan gum, pectin, dextran, xanthan gum, guar gum, carrageenan, glucomannan, and fibrin gel.

Preferably, the hydrogel 14 is a gel mainly composed of alginate gel, that is, an alginate gel. In this case, the hydrogel precursor may be a solution mainly composed of an alginate solution. The alginate gel can be formed by crosslinking an alginate solution as an alginate derivative with a divalent metal ion. The alginate gel can be suitably used as a hydrogel for culturing cells or cell aggregates in a state where the cells or cell aggregates are encapsulated.

### [First Embodiment]

In the method for producing a cell capsule, first, a core, a hydrogel precursor, a gelling solution, and a collection solution are prepared. The core may be a cell suspension. The hydrogel precursor may include a material that forms a hydrogel by being gelled. The gelling solution may contain a material that gels the hydrogel precursor.

A method for producing a cell capsule includes continuously discharging a hydrogel or a hydrogel precursor covering cells into a collection solution. The composition of the core, the hydrogel precursor, the gelling solution, and the collection solution will be described in detail below.

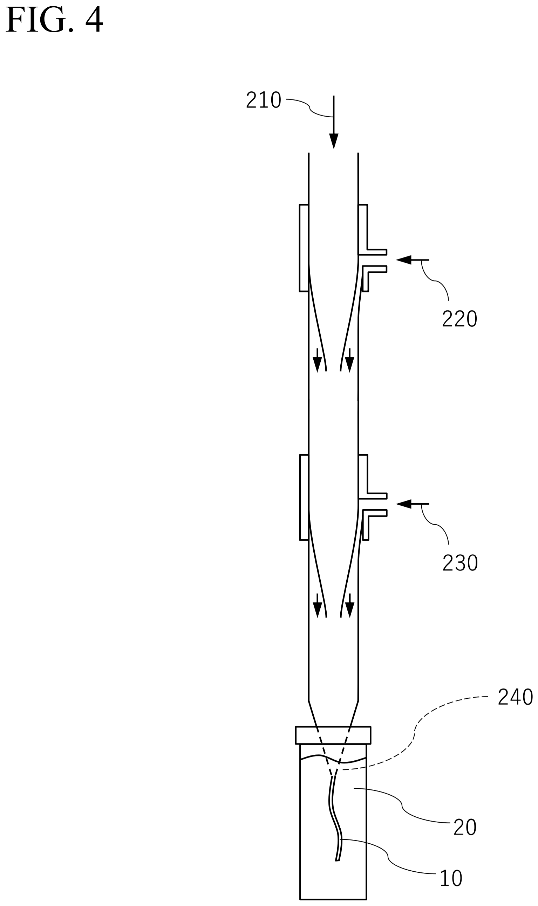

A method for producing a cell capsule according to a first embodiment will be described in more detail with reference to Fig. 4. Fig. 4 is a schematic view for describing the method for producing a cell capsule according to the first embodiment. A device for producing a cell capsule may have a first inlet 210, a second inlet 220, a third inlet 230, and an ejection port 240. The first inlet 210 is an inflow port for allowing the core to flow in. The second inlet 220 is an inflow port for allowing the hydrogel precursor to flow in. The third inlet 230 is an inflow port for allowing a solution for gelling the hydrogel precursor (gelling solution) to flow in.

The hydrogel precursor flows in from the second inlet 220 and joins around the core. Thereby, the hydrogel precursor flows around the core flow and along the core flow direction. That is, the hydrogel precursor surrounds the core in a cross section orthogonal to the flow of the core. More specifically, the hydrogel precursor and the core preferably flow to form a laminar flow.

The gelling solution is a solution for gelling the hydrogel precursor to form a hydrogel. The gelling solution is joined to the core and the hydrogel precursor on the downstream side of the junction of the core and the hydrogel precursor. The gelling solution flows along the flow of the hydrogel precursor around the hydrogel precursor surrounding the core. That is, the gelling solution surrounds the hydrogel precursor in a cross section orthogonal to the flow of the hydrogel precursor. The gelling solution preferably forms a laminar flow together with the core and the hydrogel precursor.

In the first embodiment, when the gelling solution joins the hydrogel precursor, the hydrogel precursor becomes a hydrogel. The hydrogel precursor may be completely in a hydrogel state when being discharged from the ejection port 240, or may be discharged from the ejection port 240 while remaining in a state of the precursor. Thus, the hydrogel or the hydrogel precursor covers the core containing the cells and is discharged together with the core into the collection solution 20.

In the first embodiment, the gelling solution covering the hydrogel or the hydrogel precursor is discharged into the collection solution together with the hydrogel or the hydrogel precursor.

As shown in Fig. 4, the hydrogel or the hydrogel precursor is continuously discharged into the collection solution together with the core. The hydrogel or a hydrogel generated from the hydrogel precursor is maintained in the collection solution for, for example, 0.25 hours or more, 0.5 hours or more, 1 hour or more, 2 hours or more, or 4 hours or more from the start of discharge of the hydrogel or the hydrogel precursor. The time for discharging the core, the hydrogel or the hydrogel precursor, and the gelling solution may be, for example, 0.25 hours or more, 0.5 hours or more, 1 hour or more, 2 hours or more, or 4 hours or more.

The time during which the hydrogel or the hydrogel generated from the hydrogel precursor is maintained in the collection solution is not particularly limited, but may be, for example, 24 hours or less, 20 hours or less, 16 hours or less, 12 hours or less, 8 hours or less, or 6 hours or less from the start of discharge of the hydrogel or the hydrogel precursor. In addition, the time for discharging the core, the hydrogel or the hydrogel precursor, and the gelling solution is not particularly limited, but may be, for example, 24 hours or less, 20 hours or less, 16 hours or less, 12 hours or less, 8 hours or less, or 6 hours or less.

In Fig. 4, the hydrogel or the hydrogel precursor is continuously discharged into the collection solution together with the core. The present invention is not limited thereto, and the hydrogel or the hydrogel precursor may be intermittently discharged into the collection solution together with the core.

From the viewpoint of producing a large cell population, the fluid having the highest flow rate among the core, the hydrogel precursor or the hydrogel, and the gelling solution may be flowed into the collection solution in an amount of, for example, 1 mL or more. Preferably, the core containing cells may be flowed into the collection solution in an amount of, for example, 1 mL or more.

The average temperature of the collection solution may be, for example, 40°C or lower, 30°C or lower, preferably 25°C or lower, and more preferably 20°C or lower. By lowering the average temperature of the collection solution, adverse effects on the cells can be mitigated. The average temperature can be calculated by measuring the temperature of a plurality of parts of the collection solution and dividing the temperature by the number of measured parts. In a case where the temperature of the collection solution is substantially uniform throughout the collection solution, the average temperature of the collection solution can also be determined by the temperature at one location in the collection solution.

The average temperature of the collection solution may be, for example, 0°C or higher, 2°C or higher, or 4°C or higher. The temperature of the collection solution may be in a range in which the upper limit value and the lower limit value are combined in any manner. The average temperature of the collection solution is desirably maintained in the above range while the hydrogel or the hydrogel precursor is discharged to the collection solution. Alternatively, the average temperature of the collection solution may be set to the above range immediately after the hydrogel or the hydrogel precursor finishes the discharge to the collection solution. In a case where the average temperature of the collection solution is set to the above range immediately after the hydrogel or the hydrogel precursor finishes the discharge to the collection solution, the cell capsule may be maintained in the collection solution for, for example, 0.25 hours or more, 0.5 hours or more, 1 hour or more, 2 hours or more, or 4 hours or more. The time during which the cell capsule is maintained in the collection solution is not particularly limited, but may be, for example, 24 hours or less.

The core may be a cell suspension. The core may contain at least one selected from the group consisting of physiological saline, an aqueous solution of an inorganic salt, an aqueous solution of a saccharide, a culture medium, a culture supernatant, a buffer solution, an extracellular matrix, a thickening agent, and a hydrophilic resin (such as polyvinyl alcohol, polyacrylamide, or poly(N-isopropylacrylamide)) .

The thickening agent may contain at least one selected from the group consisting of chitosan gel, collagen solution, Matrigel, collagen, gelatin, alginate solution, alginate gel, peptide gel, laminin, agarose, nanocellulose, methylcellulose, hyaluronic acid, proteoglycan, elastin, pullulan, dextran, pectin, gellan gum, xanthan gum, guar gum, carrageenan, and glucomannan.

The core may contain various growth factors suitable for cell culture, cell maintenance and proliferation, cell functional expression, or the like. Such growth factors may be at least one selected from the group consisting of epidermal growth factor (EGF), platelet-derived growth factor (PDGF), transforming growth factor (TGF), insulin-like growth factor (IGF), fibroblast growth factor (FGF), nerve growth factor (NGF), vascular endothelial growth factor (VEGF), and hepatocyte growth factor (HGF).

The type of the cell is not particularly limited. The cell may be, for example, an animal cell, and preferably a human cell. The cells may be, for example, various types of stem cells having pluripotency, human ES cells or human iPS cells having totipotency, stem cells having pluripotency, or precursor cells having oligopotency or unipotency. Examples of various stem cells having totipotency and pluripotency include iPS cells, ES cells, mesenchymal stem cells, neural stem cells, hepatic stem cells, skin stem cells, and pancreatic stem cells. Examples of stem cells or precursor cells having oligopotency or unipotency include muscle stem cells, germline stem cells, respiratory precursor cells, gastrointestinal precursor cells, and cardiac precursor cells. In addition, the adherent cells may be various types of differentiated cells, such as muscle cells including skeletal muscle cells, smooth muscle cells, and cardiomyocytes; nerve cells such as cerebral cortex cells; fibroblasts; epithelial cells; endothelial cells; adipocytes; osteoblasts; chondrocytes; macrophages; dendritic cells; hepatocytes; hepatic stellate cells; pancreatic β cells; keratinocytes; renal cells; tubular epithelial cells; hair matrix cells; corneal endothelial cells; photoreceptor cells; retinal pigment epithelial cells; goblet cells; and respiratory cells. Cells produced by differentiation induction from cells having totipotency or pluripotency may also be used. The cells may also be, for example, CHO cells or cell lines derived from human hepatocellular carcinoma. Furthermore, the cells also include tissues or organs composed of assemblies of the above-described cells, fungi such as bacteria, oomycetes, slime molds, and true fungi, and microorganisms such as algae. Examples of fungi include koji molds and yeast.

The material constituting the hydrogel precursor is appropriately selected according to the material of the hydrogel described above. That is, the hydrogel precursor may contain at least one precursor selected from the group consisting of alginate gel, Matrigel, collagen gel, chitosan gel, gelatin, peptide gel, laminin gel, agarose gel, nanocellulose, methylcellulose, dextran, pectin, gellan gum, xanthan gum, guar gum, carrageenan, glucomannan, fibrin gel, tetra-PEG gel, polyacrylamide gel, polyrotaxane, and poly(N-isopropylacrylamide) polymer.

Preferably, the hydrogel precursor may be a solution (alginate derivative) mainly composed of an alginate solution. The alginate solution may be, for example, sodium alginate, potassium alginate, or ammonium alginate, or a combination thereof. The alginate derivative may be a naturally extracted product or may be chemically modified. Examples of chemically modified alginate include methacrylate-modified alginate. The alginate solution is easily and rapidly crosslinked by divalent metal ions at room temperature or near room temperature, and tends to form an alginate gel.

The collection solution contains a pH buffer. As a result, the hydrogen ion index of the collection solution is stably maintained. In the first embodiment, the hydrogel, the hydrogel precursor and/or the gelling solution flow into the collection solution during the production of the cell capsule. In particular, in the case of producing long cell capsules, the hydrogel, hydrogel precursor, and/or gelling solution are discharged into the collection solution for a long period of time. Even in such a case, when the collection solution contains a pH buffer, the hydrogen ion index of the collection solution is stably maintained over a long period of time. Accordingly, it is possible to mitigate adverse effects on the cells in the cell capsule in the collection solution.

The collection solution may be, for example, an acetic acid buffer, a phosphate buffer, a citric acid buffer, a citrate-phosphate buffer, a boric acid buffer, a tartaric acid buffer, phosphate-buffered saline, or a so-called Good's buffer. Preferably, the pH buffer in the collection solution is a Good's buffer. It is considered that some Good's buffers can be suitably used even when mixed with a gelling solution because the buffers are difficult to form a complex with metal ions.

The Good's buffer may contain at least one selected from the group consisting of ACES (N-(2-acetamido)-2-aminoethanesulfonic acid), ADA (N-(2-acetamido)iminodiacetic acid), BES (N,N-bis(2-hydroxyethyl)-2-aminoethanesulfonic acid), Bicine (N,N-bis (2-hydroxyethyl) glycine), Bis-Tris (bis(2-hydroxyethyl)iminotris(hydroxymethyl)methane), CAPS (3-(cyclohexylamino)propanesulfonic acid), CAPSO (N-cyclohexyl-2-hydroxy-3-aminopropanesulfonic acid), CHES (2-(cyclohexylamino)ethanesulfonic acid), DIPSO (3-[N,N-bis(2-hydroxyethyl)amino]-2-hydroxypropanesulfonic acid), EPPS (4-(2-hydroxyethyl)-1-piperazine-1-propanesulfonic acid), HEPES (2-[4-(2-hydroxyethyl)-1-piperazinyl]ethanesulfonic acid), HEPES-Na (2-[4-(2-hydroxyethyl)-1-piperazinyl]ethanesulfonic acid sodium salt), HEPPSO (4-(2-hydroxyethyl)piperazine-1-(2-hydroxypropane-3-sulfonic acid)), MES (2-morpholinoethanesulfonic acid monohydrate), MOPS (3-morpholinopropanesulfonic acid), MOPSO (2-hydroxy-3-morpholinopropanesulfonic acid), PIPES (piperazine-1,4-bis(2-ethanesulfonic acid)), PIPES sodium salt (piperazine-1,4-bis(2-ethanesulfonic acid) sodium salt), POPSO (piperazine-1,4-bis(2-hydroxypropanesulfonic acid)), TAPS (N-[tris(hydroxymethyl)methyl]-3-aminopropanesulfonic acid), TAPSO (3-[N-tris(hydroxymethyl)methylamino]-2-hydroxypropanesulfonic acid), TES (N-tris(hydroxymethyl)methyl-2-aminoethanesulfonic acid), and Tricine (N-[tris(hydroxymethyl)methyl]glycine).

The collection solution may contain nutrients such as a carbon source, vitamins, and inorganic salts. As such an example, the collection solution may contain a culture medium having buffering capacity. The type of the culture medium may be appropriately selected according to the type of cells to be housed inside the tubular hydrogel.

The collection solution preferably contains a gelling agent for gelling the hydrogel precursor. The gelling agent may be any material capable of forming a hydrogel by crosslinking the hydrogel precursor. For example, when the hydrogel precursor is alginate, the gelling agent may be a material capable of providing divalent cations. Examples of such a gelling agent include salts of magnesium, calcium, strontium or barium. Preferably, the gelling agent may be, for example, calcium chloride, calcium carbonate, calcium hydroxide, barium chloride, barium carbonate, or barium hydroxide. That is, the collection solution may contain divalent cations such as calcium ions or barium ions.

The concentration of the gelling material, for example, the concentration of calcium chloride or barium chloride, may be, for example, 0 to 500 mmol/L, and preferably 5 to 200 mmol/L.

When the collection solution contains a gelling agent, it is preferable that the buffer is composed of one that is not substantially precipitated by the gelling agent. When the collection solution contains a gelling agent, even in a case where the hydrogel precursor is discharged into the collection solution before becoming a hydrogel, the hydrogel precursor can become a hydrogel in the collection solution.

The pH of the collection solution may be, for example, in the range of 6.5 to 8.0, and preferably 6.8 to 7.5. When the pH is in this range, adverse effects on cells can be further suppressed. When the collection solution contains a pH buffer, the pH of the collection solution is likely to be maintained in the range of, for example, 6.5 to 8.0, and preferably 6.8 to 7.5, during the time of discharge of the hydrogel precursor or hydrogel and/or during the time of incubation of the cells in the cell capsule.

The osmotic pressure of the collection solution may be, for example, in the range of 100 to 1,500 mOsm, and preferably in the range of 200 to 400 mOsm. When the osmotic pressure is in this range, adverse effects on cells can be further suppressed.

The gelling solution may be a solution containing a gelling agent. The gelling agent may be any material capable of forming a hydrogel by crosslinking the hydrogel precursor. For example, when the hydrogel precursor is alginate, the gelling agent may be a material capable of providing divalent cations. Examples of such a gelling agent include salts of magnesium, calcium, strontium or barium. Preferably, the gelling agent may be, for example, calcium chloride, calcium carbonate, calcium hydroxide, barium chloride, barium carbonate, or barium hydroxide. That is, the gelling solution may contain divalent cations such as calcium ions and barium ions.

The concentration of divalent metal ions in the gelling solution, such as the concentration of calcium chloride or barium chloride, may be, for example, 10 to 500 mmol/L, and more preferably 20 to 200 mmol/L.

The gelling solution preferably contains a pH buffer. In this case, the pH buffer is more preferably a Good's buffer. As the pH buffer, those listed above can be used. In this case, even when a large amount of the gelling solution flows into the collection solution, the hydrogen ion index in the collection solution is likely to be stably maintained. Therefore, adverse effects on the cells in the cell capsules in the collection solution can be further mitigated.

The gelling solution may contain nutrients such as a carbon source, vitamins, and inorganic salts. As such an example, the gelling solution may contain a culture medium having buffering capacity. The type of the culture medium may be appropriately selected according to the type of cells to be housed inside the tubular hydrogel.

The pH of the gelling solution may be, for example, in the range of 6.5 to 8.0, and preferably 6.8 to 7.5. When the pH is in this range, adverse effects on cells can be further suppressed.

The osmotic pressure of the gelling solution may be, for example, in the range of 100 to 1,500 mOsm, and preferably in the range of 200 to 400 mOsm. When the osmotic pressure is in this range, adverse effects on cells can be further suppressed.

The gelling solution preferably has the same pH buffer as that included in the collection solution. In this case, even when a large amount of the gelling solution flows into the collection solution, the hydrogen ion index in the collection solution is likely to be stably maintained. The gelling solution more preferably has the same composition as the collection solution. In this case, even when a large amount of the gelling solution flows into the collection solution, the hydrogen ion index in the collection solution is likely to be more stably maintained.

### [Second Embodiment]

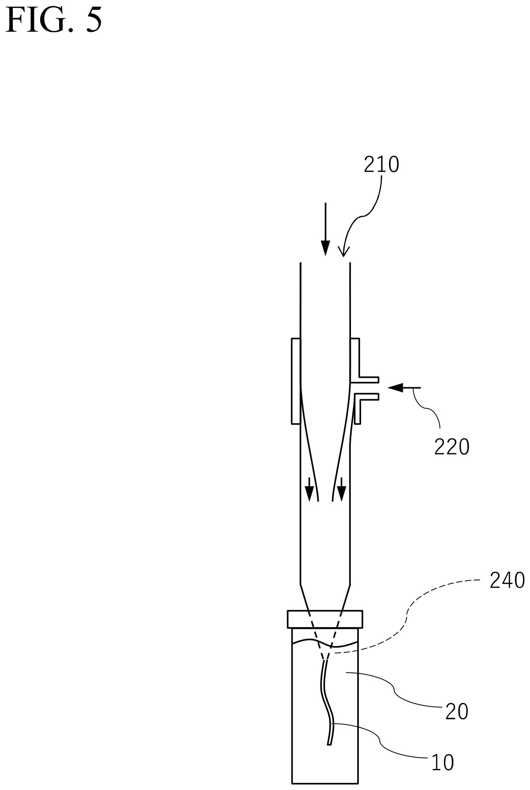

A method for producing a cell capsule according to a second embodiment will be described. Fig. 5 is a schematic view for describing the method for producing a cell capsule according to the second embodiment. In the second embodiment, the description of the same configuration and/or process as those of the first embodiment may be omitted.

First, a core, a hydrogel precursor, a gelling solution, and a collection solution are prepared. Materials, cells, and the like constituting the core, the hydrogel precursor, and the collection solution are the same as those in the first embodiment.

In the second embodiment, as shown in Fig. 5, a device for producing a cell capsule may have the first inlet 210, the second inlet 220, and the ejection port 240. The first inlet 210 is an inflow port for allowing the core to flow in. The second inlet 220 is an inflow port for allowing the hydrogel precursor to flow in. In the second embodiment, the ejection port 240 is immersed in the collection solution.

The hydrogel precursor flows in from the second inlet 220 and joins around the core. Thereby, the hydrogel precursor flows around the core flow and along the core flow direction. That is, the hydrogel precursor surrounds the core in a cross section orthogonal to the flow of the core. More specifically, the hydrogel precursor and the core preferably flow to form a laminar flow.

In the second embodiment, the collection solution has a gelling agent for gelling the hydrogel precursor. In the second embodiment, the hydrogel precursor covers the core containing the cells and is continuously discharged into the collection solution together with the core. The hydrogel precursor is gelled in the collection solution to form a hydrogel. Accordingly, a long tubular hydrogel covering the core containing cells is formed.

As shown in Fig. 5, the hydrogel precursor is continuously discharged into the collection solution together with the core. The core discharged together with the hydrogel precursor is maintained in the collection solution for, for example, 0.25 hours or more, 0.5 hours or more, 1 hour or more, 2 hours or more, or 4 hours or more from the start of discharge of the hydrogel precursor. The time for discharging the core and the hydrogel precursor may be, for example, 0.25 hours or more, 0.5 hours or more, 1 hour or more, 2 hours or more, or 4 hours or more.

The time during which the core discharged together with the hydrogel precursor is maintained in the collection solution is not particularly limited, but may be, for example, 24 hours or less, 20 hours or less, 16 hours or less, 12 hours or less, 8 hours or less, or 6 hours or less from the start of discharge of the core and the hydrogel precursor. The time for discharging the core and the hydrogel precursor is not particularly limited, but may be, for example, 24 hours or less, 20 hours or less, 16 hours or less, 12 hours or less, 8 hours or less, or 6 hours or less.

The average temperature of the collection solution may be, for example, 40°C or lower, 30°C or lower, preferably 25°C or lower, and more preferably 20°C or lower. By lowering the average temperature of the collection solution, adverse effects on the cells can be mitigated.

The average temperature of the collection solution may be, for example, 0°C or higher, 2°C or higher, or 4°C or higher. The average temperature of the collection solution may be in a range in which the upper limit value and the lower limit value are combined in any manner. The average temperature of the collection solution is desirably maintained in the above range while the hydrogel precursor is discharged to the collection solution. Alternatively, the average temperature of the collection solution may be set to the above range immediately after the hydrogel precursor finishes the discharge to the collection solution. In a case where the average temperature of the collection solution is set to the above range immediately after the hydrogel precursor finishes the discharge to the collection solution, the cell capsule may be maintained in the collection solution for, for example, 0.25 hours or more, 0.5 hours or more, 1 hour or more, 2 hours or more, or 4 hours or more. The time during which the cell capsule is maintained in the collection solution is not particularly limited, but may be, for example, 24 hours or less.

From the viewpoint of producing a large cell population, the fluid having the higher flow rate among the core and the hydrogel precursor may be flowed into the collection solution in an amount of, for example, 1 mL or more. Preferably, the core containing cells may be flowed into the collection solution in an amount of, for example, 1 mL or more.

Also in the second embodiment, it is possible to produce a long cell capsule as described in Figs. 1 and 2.

### [Third Embodiment]

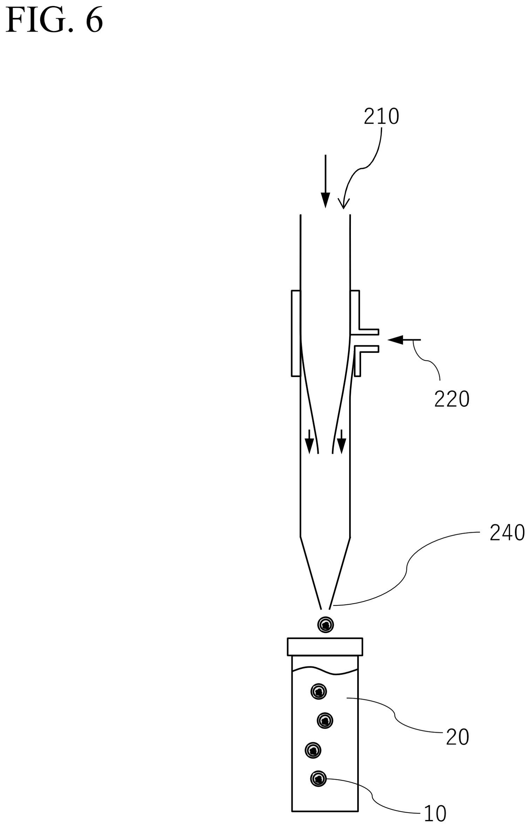

A method for producing a cell capsule according to a third embodiment will be described. Fig. 6 is a schematic view for describing the method for producing a cell capsule according to the third embodiment. In the third embodiment, the description of the same configuration and/or process as those of the first and second embodiments may be omitted.

First, a core, a hydrogel precursor, a gelling solution, and a collection solution are prepared. Materials, cells, and the like constituting the core, the hydrogel precursor, and the collection solution are the same as those in the first and second embodiments.

In the third embodiment, as shown in Fig. 6, a device for producing a cell capsule may have, similarly to the second embodiment, the first inlet 210, the second inlet 220, and the ejection port 240. The first inlet 210 is an inflow port for allowing the core to flow in. The second inlet 220 is an inflow port for allowing the hydrogel precursor to flow in. In the third embodiment, the ejection port 240 is positioned above the collection solution.

In the third embodiment, the collection solution has a gelling agent for gelling the hydrogel precursor. In the third embodiment, the hydrogel precursor covers the core containing the cells and is intermittently discharged into the collection solution together with the core. At this time, the hydrogel precursor and the core become substantially spherical droplets before reaching the collection solution from the ejection port 240. The hydrogel precursor and the core are introduced into the collection solution in the form of substantially spherical droplets. The hydrogel precursor is gelled in the collection solution to form a hydrogel. Thus, a cell capsule containing a substantially spherical core containing cells and a substantially spherical shell-like hydrogel covering the core is produced.

As shown in Fig. 6, the hydrogel precursor is intermittently discharged into the collection solution together with the core. The core discharged together with the hydrogel precursor is maintained in the collection solution for, for example, 0.25 hours or more, 0.5 hours or more, 1 hour or more, 2 hours or more, or 4 hours or more from the start of discharge of the hydrogel precursor. The time for discharging the core and the hydrogel precursor may be, for example, 0.25 hours or more, 0.5 hours or more, 1 hour or more, 2 hours or more, or 4 hours or more. As a result, a large number of spherical cell capsules are obtained.

The time during which the core discharged together with the hydrogel precursor is maintained in the collection solution is not particularly limited, but may be, for example, 24 hours or less, 20 hours or less, 16 hours or less, 12 hours or less, 8 hours or less, or 6 hours or less from the start of discharge of the hydrogel precursor. The time for discharging the core and the hydrogel precursor is not particularly limited, but may be, for example, 24 hours or less, 20 hours or less, 16 hours or less, 12 hours or less, 8 hours or less, or 6 hours or less.

The average temperature of the collection solution may be, for example, 40°C or lower, 30°C or lower, preferably 25°C or lower, and more preferably 20°C or lower. By lowering the average temperature of the collection solution, adverse effects on the cells can be mitigated.

The average temperature of the collection solution may be, for example, 0°C or higher, 2°C or higher, or 4°C or higher. The average temperature of the collection solution may be in a range in which the upper limit value and the lower limit value are combined in any manner. The average temperature of the collection solution is desirably maintained in the above range while the hydrogel precursor is discharged to the collection solution. Alternatively, the average temperature of the collection solution may be set to the above range immediately after the hydrogel precursor finishes the discharge to the collection solution. In a case where the average temperature of the collection solution is set to the above range immediately after the hydrogel precursor finishes the discharge to the collection solution, the cell capsule may be maintained in the collection solution for, for example, 0.25 hours or more, 0.5 hours or more, 1 hour or more, 2 hours or more, or 4 hours or more. The time during which the cell capsule is maintained in the collection solution is not particularly limited, but may be, for example, 24 hours or less.

From the viewpoint of producing a large cell population, the fluid having the higher flow rate among the core and the hydrogel precursor may be flowed into the collection solution in an amount of, for example, 1 mL or more. Preferably, the core containing cells may be flowed into the collection solution in an amount of, for example, 1 mL or more.

In the plurality of embodiments described above, the method for producing a cell capsule having a core containing cells and a hydrogel covering the core has been described. However, it should be noted that the cell capsule produced by the production method of the present invention is not limited thereto. For example, the cell capsule may have a structure including a hydrogel in which cells are embedded. This can be achieved, for example, by the above core containing both the hydrogel precursor and cells. Even in this case, when the collection solution that collects the hydrogel precursor or the hydrogel during the production of the cell capsules contains a pH buffer, adverse effects on the cells can be mitigated.

The cell capsule produced by the method for producing a cell capsule is used, for example, for culturing cells. In this case, the cells are cultured by being immersed in the culture medium together with the cell capsules. Thereby, the cells can be cultured inside the hydrogel.

The cell population of the present invention may be a cell population produced by the cell culture method described above. Specifically, in one example, the cell population is formed by recovering cells from the inside of the hydrogel. The recovery of cells can be achieved, for example, by dissolving and removing the hydrogel. The hydrogel can be removed by a chemical reaction, an enzymatic reaction, or the like. Specifically, the hydrogel can be removed by, for example, an EDTA/PBS solution or an alginate lyase, or a combination thereof.

The cell or the cell population may produce a cell product in a state covered with the cell capsule. This cell product can be produced, for example, by recovering the cell product from a culture medium in which the cell or the cell population has been cultured. The cell product is not particularly limited, but may be, for example, a protein, extracellular vesicle, antibody, or the like.

### [Example 1]

Example 1 will be described below. First, a core, a hydrogel precursor, a gelling solution, and a collection solution were prepared. The core is a cell suspension. The cell suspension was prepared by suspending human iPS cells (QHJI01s04) in Iscove's Modified Dulbecco's Medium containing 3% methylcellulose (R&D systems HSC001) with the addition of 0.6 w/v% methylcellulose. The initial cell density in the cell suspension was 1 x 10⁷ cells/mL.

The hydrogel precursor is a sodium alginate solution. The sodium alginate solution was generated by adding the above sodium alginate ("High-G ALG300" manufactured by KIMICA Corporation) to physiological saline and stirring. The concentration of sodium alginate relative to saline was 1.0 w/v% percent concentration.

The gelling solution was an aqueous solution containing 100 mM calcium chloride and 3 w/v% sucrose.

Using these cores, hydrogel precursors, and gelling solutions, a cell population was produced based on the method for producing a cell capsule according to the first embodiment (refer to Fig. 4). That is, a flow of the core, a flow of the hydrogel precursor around the flow of the core, and a flow of the gelling solution around the flow of the hydrogel precursor were formed, and these flows were discharged from the ejection port 240 into the collection solution.

The hydrogel precursor was crosslinked by contacting the gelling solution to form a tubular alginate gel. Thereby, a hydrogel covering the core containing the cells was produced in the collection solution.

In Example 1, the collection solution was obtained by adding a 25mM HEPES buffer and a ROCK inhibitor (Y-27632) to a 100 mM calcium chloride aqueous solution. The ROCK inhibitor was added to a concentration of 10 µM relative to the final solvent.

The flow speed of the core was about 50 µL/min, and the core was flowed for about 1 minute. Thus, the amount of core housed inside the tubular alginate gel was about 50 µL. After the core, the alginate gel, and the gelling solution were discharged into the collection solution, the lid of the container housing the collection solution was immediately closed, and the container was incubated at a predetermined time and temperature. In Example 1, the incubation temperature is 4°C and the incubation time is 1 hour (refer to Table 1 below). During this incubation, the average temperature of the collection solution is maintained at the incubation temperature (4°C in Example 1).

After incubating the cell capsules in the collection solution, the cell capsules were removed from the collection solution and immersed in the culture medium, and cell culture was started. That is, the cells were cultured while being housed inside the tubular hydrogel. The culture medium is one to which 0.1 v/v% gentamicin sulfate solution (manufactured by FUJIFILM Wako Pure Chemical Corporation) and 10 µM ROCK inhibitor (Y-27632) have been added to "mTeSR-plus (manufactured by STEMCELL Technologies)".

When the day of starting cell culture was designated as "Day 0", the entire amount of the culture medium was replaced on Day 2, Day 5, and Day 6. The new culture medium is similar to the culture medium used on Day 0.

Cells were recovered from cell capsules on Day7. Using the cell counting device NC-200 (automated cell counter; manufactured by M&S TechnoSystems, Inc.), the number of live cells in the recovered cells was measured, and the proliferation rate was calculated by dividing the total number of live cells by the initial cell number on Day 0.

The supernatant of the culture medium on Day 7 was recovered, and the lactate amount in the supernatant was measured. The lactate amount was measured using a cell culture medium analyzing device (manufactured by Nova Biomedical; Prime).

### [Example 2]

Example 2 will be described below. The cell capsule according to Example 2 was produced in the same manner as in Example 1 except for the incubation temperature before the start of culture. Specifically, in Example 2, after the core, the alginate gel, and the gelling solution were discharged into the collection solution, the lid of the container housing the collection solution was immediately closed, and the container was incubated at 20°C for 1 hour (refer to Table 1 below). Culture of cells after incubation was performed in the same manner as in Example 1.

### [Example 3]

Example 3 will be described below. The cell capsule according to Example 3 was produced in the same manner as in Example 1 except for the incubation time before the start of culture. Specifically, in Example 3, after the core, the alginate gel, and the gelling solution were discharged into the collection solution, the lid of the container housing the collection solution was immediately closed, and the container was incubated at 4°C for 2 hours (refer to Table 1 below). Culture of cells after incubation was performed in the same manner as in Example 1.

### [Example 4]

Example 4 will be described below. The cell capsule according to Example 4 was produced in the same manner as in Example 1 except for the incubation time and the incubation temperature before the start of culture. Specifically, in Example 4, after the core, the alginate gel, and the gelling solution were discharged into the collection solution, the lid of the container housing the collection solution was immediately closed, and the container was incubated at 20°C for 2 hours (refer to Table 1 below). Culture of cells after incubation was performed in the same manner as in Example 1.

### [Example 5]

Example 5 will be described below. The cell capsule according to Example 5 was produced in the same manner as in Example 1 except for the incubation time before the start of culture. Specifically, in Example 5, after the core, the alginate gel, and the gelling solution were discharged into the collection solution, the lid of the container housing the collection solution was immediately closed, and the container was incubated at 4°C for 3 hours (refer to Table 1 below). Culture of cells after incubation was performed in the same manner as in Example 1.

### [Example 6]

Example 6 will be described below. The cell capsule according to Example 6 was produced in the same manner as in Example 1 except for the incubation time and the incubation temperature before the start of culture. Specifically, in Example 6, after the core, the alginate gel, and the gelling solution were discharged into the collection solution, the lid of the container housing the collection solution was immediately closed, and the container was incubated at 20°C for 3 hours (refer to Table 1 below). Culture of cells after incubation was performed in the same manner as in Example 1.

### [Reference Examples 1 to 6]

Reference Examples 1 to 6 will be described below. The cell capsules according to Reference Examples 1 to 6 were produced in the same manner as Examples 1 to 6, respectively, except for the composition of the collection solution. The collection solution according to Reference Examples 1 to 6 is one in which 10 µM ROCK inhibitor (Y-27632) was added to an aqueous solution of 100 mM calcium chloride. Incubation of cell capsules in the collection solution and culture of cells after incubation were performed in the same manner as in Examples 1 to 6 (refer to Table 1 below).

**(Table 1)**

| | Cell | Composition of Collection Solution | Incubation temperature before starting the culture | Incubation time before starting the culture |
|---|---|---|---|---|
| Example 1 | iPS Cells | CaCl₂ + HEPES + ROCK inhibitor | 4°C | 1 hour |
| Example 2 | iPS Cells | CaCl₂ + HEPES + ROCK inhibitor | 20°C | 1 hour |
| Example 3 | iPS Cells | CaCl₂ + HEPES + ROCK inhibitor | 4°C | 2 hours |
| Example 4 | iPS Cells | CaCl₂ + HEPES + ROCK inhibitor | 20°C | 2 hours |
| Example 5 | iPS Cells | CaCl₂ + HEPES + ROCK inhibitor | 4°C | 3 hours |
| Example 6 | iPS Cells | CaCl₂ + HEPES + ROCK inhibitor | 20°C | 3 hours |
| Reference Example 1 | iPS Cells | CaCl₂ + ROCK inhibitor | 4°C | 1 hour |
| Reference Example 2 | iPS Cells | CaCl₂ + ROCK inhibitor | 20°C | 1 hour |
| Reference Example 3 | iPS Cells | CaCl₂ + ROCK inhibitor | 4°C | 2 hours |
| Reference Example 4 | iPS Cells | CaCl₂ + ROCK inhibitor | 20°C | 2 hours |
| Reference Example 5 | iPS Cells | CaCl₂ + ROCK inhibitor | 4°C | 3 hours |
| Reference Example 6 | iPS Cells | CaCl₂ + ROCK inhibitor | 20°C | 3 hours |

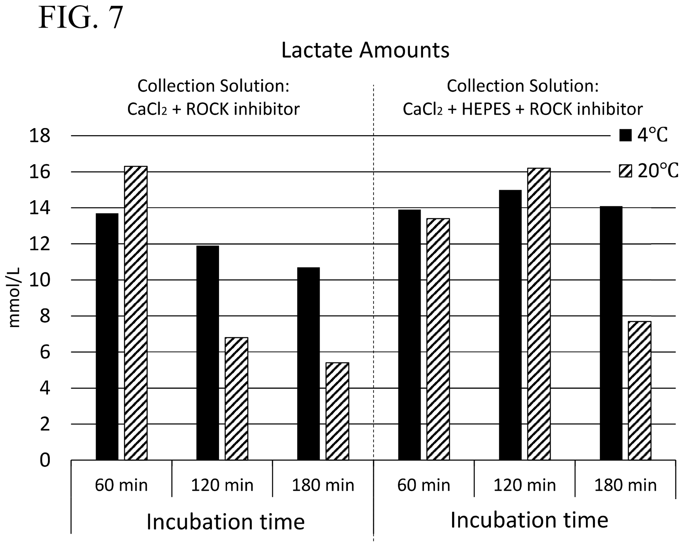

Fig. 7 is a graph showing measurement results of a lactate amount in a culture medium on Day 7 in Reference Examples 1 to 6 and Examples 1 to 6. In Fig. 7, the lactate amounts measured in Reference Example 1, Reference Example 2, Reference Example 3, Reference Example 4, Reference Example 5, Reference Example 6, Example 1, Example 2, Example 3, Example 4, Example 5, and Example 6 are shown in order from the left.

Referring to Fig. 7, it can be seen that, in Reference Examples 1 to 6, the lactate amount in the culture medium on Day 7 decreases as the incubation time becomes longer. In particular, in a case where the incubation temperature is 20°C, which is relatively high, the lactate amount in the culture medium on Day 7 decreases significantly as the incubation time becomes longer. It is considered that, when the cell capsules are immersed in the collection solution for a long time before cell culture, this adversely affects cell culture and suppresses cell proliferation, resulting in a decrease in the lactate amount, which is a metabolite in cells.

In a case where the average temperature of the collection solution is 4°C as in Examples 1, 3, and 5, the lactate amount in the culture medium on Day 7 is maintained at a high value even when the incubation time becomes longer. In a case where the average temperature of the collection solution is 20°C as in Examples 2, 4, and 6, when the incubation time is 2 hours or less, the lactate amount in the culture medium on Day 7 is maintained at a high value. Comparing Reference Examples 3 to 6 with Examples 3 to 6, when the incubation time is 2 to 3 hours, the lactate amount in Examples 3 to 6 is higher.

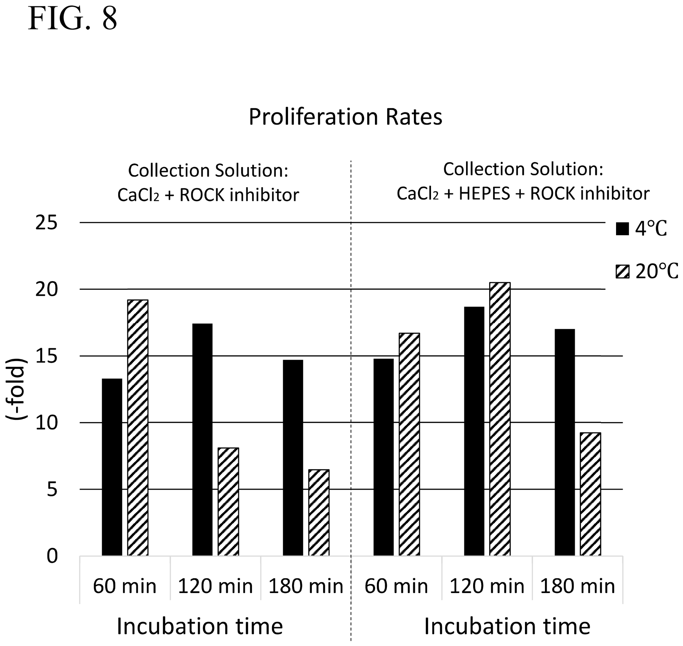

Fig. 8 is a graph showing measurement results of a proliferation rate of cells on Day 7 in Reference Examples 1 to 6 and Examples 1 to 6. In Fig. 8, the proliferation rates of cells measured in Reference Example 1, Reference Example 2, Reference Example 3, Reference Example 4, Reference Example 5, Reference Example 6, Example 1, Example 2, Example 3, Example 4, Example 5, and Example 6 are shown in order from the left.

Referring to Fig. 8, it can be seen that, in Reference Examples 1 to 6, the proliferation rate of cells generally decreases as the incubation time becomes longer. In particular, in a case where the incubation temperature is 20°C, which is relatively high, the proliferation rate of cells decreases significantly as the incubation time becomes longer. This result is substantially consistent with the measurement result of the lactate amount described above. That is, it can be seen that, when the cell capsule is immersed in the collection solution for a long time before the cell is cultured, the cell culture is adversely affected.

In a case where the average temperature of the collection solution is 4°C as in Examples 1, 3, and 5, the culture rate of cells is maintained at a high value even when the incubation time becomes longer. In a case where the average temperature of the collection solution is 20°C as in Examples 2, 4, and 6, when the incubation time is 2 hours or less, the culture rate of cells is maintained at a high value. Comparing Reference Examples 3 to 6 with Examples 3 to 6, when the incubation time is 2 to 3 hours, the culture rate of cells in Examples 3 to 6 is higher.

The difference between Examples 1 to 6 and Reference Examples 1 to 6 is in the composition of the collection solution. In Examples 1 to 6, the collection solution contains HEPES, which is a Good's buffer. As a result, it is considered that the hydrogen ion index in the collection solution was relatively stably maintained, and adverse effects on cell culture are mitigated.

### [Example 7]

Example 7 will be described below. The cell capsule according to Example 7 was produced in the same manner as in Example 1 except for the composition of the collection solution. The collection solution according to Example 7 was obtained by adding calcium chloride and a ROCK inhibitor (Y-27632) to a culture medium having pH buffering capacity. The culture medium is Dulbecco's Modified Eagle Medium (DMEM/F-12). Calcium chloride was dissolved in the culture medium to a concentration of 100 mM. ROCK inhibitor (Y-27632) was added to a concentration of 10 µM relative to the final solvent. Incubation of cell capsules in the collection solution and culture of cells after incubation were performed in the same manner as in Example 1 (refer to Table 2 below).

### [Example 8]

Example 8 will be described below. The cell capsule according to Example 8 was produced in the same manner as in Example 7 except for the incubation temperature before the start of culture. Specifically, in Example 8, after the core, the alginate gel, and the gelling solution were discharged into the collection solution, the lid of the container housing the collection solution was immediately closed, and the container was incubated at 20°C for 1 hour (refer to Table 2 below). Culture of cells after incubation was performed in the same manner as in Example 7.

### [Example 9]

Example 9 will be described below. The cell capsule according to Example 9 was produced in the same manner as in Example 7 except for the incubation time before the start of culture. Specifically, in Example 9, after the core, the alginate gel, and the gelling solution were discharged into the collection solution, the lid of the container housing the collection solution was immediately closed, and the container was incubated at 4°C for 3 hours (refer to Table 2 below). Culture of cells after incubation was performed in the same manner as in Example 7.

### [Example 10]

Example 10 will be described below. The cell capsule according to Example 10 was produced in the same manner as in Example 7 except for the incubation time and the incubation temperature before the start of culture. Specifically, in Example 10, after the core, the alginate gel, and the gelling solution were discharged into the collection solution, the lid of the container housing the collection solution was immediately closed, and the container was incubated at 20°C for 3 hours (refer to Table 2 below). Culture of cells after incubation was performed in the same manner as in Example 7.

### [Reference Examples 7 to 10]

Reference Examples 7 to 10 will be described below. The cell capsules according to Reference Examples 7 to 10 were produced in the same manner as Examples 7 to 10, respectively, except for the composition of the collection solution. The collection solution according to Reference Examples 7 to 10 is one in which ROCK inhibitor (Y-27632) was added to an aqueous solution of 100 mM calcium chloride. ROCK inhibitor (Y-27632) was added to a concentration of 10 µM relative to the final solvent. Incubation of cell capsules in the collection solution and culture of cells after incubation were performed in the same manner as in Examples 7 to 10 (refer to Table 2 below).

**(Table 2)**

| | Cell | Composition of Collection Solution | Incubation temperature before starting the culture | Incubation time before starting the culture |
|---|---|---|---|---|
| Example 7 | iPS Cells | CaCl₂ + DMEM/F-12 culture medium (including buffer) + Sucrose + ROCK inhibitor | 4°C | 1 hour |
| Example 8 | iPS Cells | CaCl₂ + DMEM/F-12 culture medium (including buffer) + Sucrose + ROCK inhibitor | 20°C | 1 hour |
| Example 9 | iPS Cells | CaCl₂ + DMEM/F-12 culture medium (including buffer) + Sucrose + ROCK inhibitor | 4°C | 3 hours |
| Example 10 | iPS Cells | CaCl₂ + DMEM/F-12 culture medium (including buffer) + Sucrose + ROCK inhibitor | 20°C | 3 hours |
| Reference Example 7 | iPS Cells | CaCl₂ + Sucrose + ROCK inhibitor | 4°C | 1 hour |
| Reference Example 8 | iPS Cells | CaCl₂ + Sucrose + ROCK inhibitor | 20°C | 1 hour |
| Reference Example 9 | iPS Cells | CaCl₂ + Sucrose + ROCK inhibitor | 4°C | 3 hours |
| Reference Example 10 | iPS Cells | CaCl₂ + Sucrose + ROCK inhibitor | 20°C | 3 hours |

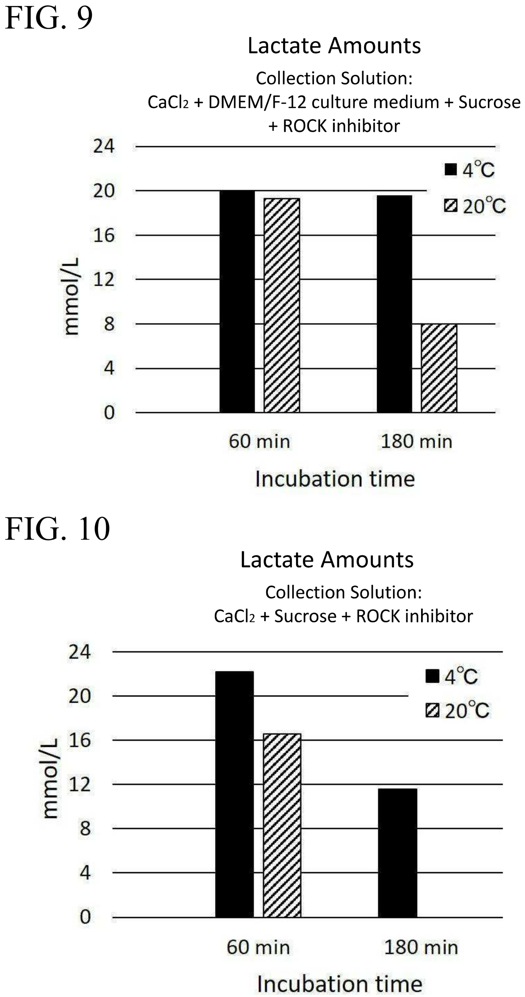

Fig. 9 is a graph showing measurement results of the lactate amount in the culture medium on Day 7 in Examples 7 to 10. The lactate amounts measured in Examples 7, 8, 9, and 10 are shown in order from the left. Fig. 10 is a graph showing measurement results of the lactate amount in the culture medium on Day 7 in Reference Examples 7 to 10. In Fig. 10, the lactate amounts measured in Reference Example 7, Reference Example 8, Reference Example 9, and Reference Example 10 are shown in order from the left.

Referring to Fig. 9, in a case where the average temperature of the collection solution is 4°C as in Examples 7 and 9, the lactate amount in the culture medium on Day 7 is maintained at a high value even when the incubation time becomes longer. In a case where the average temperature of the collection solution is 20°C as in Examples 8 and 10, when the incubation time is 1 hour, the lactate amount in the culture medium on Day 7 is maintained at a high value.

Referring to Fig. 10, it can be seen that, in Reference Examples 7 to 10, the lactate amount in the culture medium on Day 7 decreases significantly as the incubation time becomes longer. In a case where the incubation temperature was relatively high at 20°C and the incubation time was 3 hours, the number of live cells could not be maintained.

Comparing Reference Examples 7 to 10 with Examples 7 to 10, it can be observed that, in Examples 7 to 10, the decrease in lactate amount is mitigated, and the adverse effects on cells tend to be mitigated. This is considered to be because, in Examples 7 to 10, the collection solution was a culture medium having buffering capacity, and the hydrogen ion index in the collection solution was maintained relatively stably.

### [Example 11]

Example 11 will be described below. First, a core, a hydrogel precursor, a gelling solution, and a collection solution were prepared. The core is a cell suspension. The cell suspension was prepared by suspending cells (human chronic myelogenous leukemia-derived K562 cells) in Iscove's Modified Dulbecco's Medium containing 3% methylcellulose (R&D systems HSC001), to which 0.3 w/v% methylcellulose was added. The initial cell density in the cell suspension was 1 x 10⁷ cells/mL.

The hydrogel precursor is a sodium alginate solution. The sodium alginate solution was generated by adding the sodium alginate ("High-G ALG300" manufactured by KIMICA Corporation) to physiological saline and stirring. The concentration of sodium alginate relative to saline was 1.0 w/v% percent concentration.

The gelling solution was an aqueous solution containing 100 mM calcium chloride and 3 w/v% sucrose.

Using these cores, hydrogel precursors, and gelling solutions, a cell population was produced based on the method for producing a cell capsule according to the first embodiment (refer to Fig. 4). That is, a flow of the core, a flow of the hydrogel precursor around the flow of the core, and a flow of the gelling solution around the flow of the hydrogel precursor were formed, and these flows were discharged from the ejection port 240 into the collection solution.

The hydrogel precursor was crosslinked by contacting the gelling solution to form a tubular alginate gel. Thereby, a hydrogel covering the core containing the cells was produced in the collection solution.

In Example 11, the collection solution is a culture medium having pH buffering capacity. The culture medium is one in which 10% fetal bovine serum (FBS) and 0.1% gentamicin sulfate solution (manufactured by FUJIFILM Wako Pure Chemical Corporation) were added to Iscove's Modified Dulbecco's Medium containing 3% methylcellulose (R&D Systems HSC001). The flow speed of the core was about 50 µL/min, and the core was flowed for about 1 minute. Thus, the amount of core housed inside the tubular alginate gel was about 50 µL.

After the core, the alginate gel, and the gelling solution were discharged into the collection solution, the lid of the container housing the collection solution was immediately closed, and the container was incubated at a predetermined time and temperature. In Example 11, the incubation temperature is 4°C and the incubation time is 1 hour (refer to Table 3 below).

After incubating the cell capsules in the collection solution, the cell capsules were removed from the collection solution and immersed in the culture medium, and cell culture was started. That is, the cells were cultured while being housed inside the tubular hydrogel. The culture medium is one in which 10% fetal bovine serum (FBS) and 0.1% gentamicin sulfate solution (manufactured by FUJIFILM Wako Pure Chemical Corporation) were added to Iscove's Modified Dulbecco's Medium containing 3% methylcellulose (R&D Systems HSC001).

When the day of starting cell culture was designated as "Day 0", cells were recovered from the cell capsules on Day 2. The supernatant of the culture medium on Day 2 was recovered, and the lactate amount in the supernatant was measured in the same manner as in Example 1. In this manner, the lactate amount generated from Day 0 to Day 2 was measured.

### [Example 12]

Example 12 will be described below. The cell capsule according to Example 12 was produced in the same manner as in Example 11 except for the incubation temperature before the start of culture. Specifically, in Example 12, after the core, the alginate gel, and the gelling solution were discharged into the collection solution, the lid of the container housing the collection solution was immediately closed, and the container was incubated at 20°C for 1 hour (refer to Table 3 below). Culture of cells after incubation was performed in the same manner as in Example 11.

### [Example 13]

Example 13 will be described below. The cell capsule according to Example 13 was produced in the same manner as in Example 11 except for the incubation temperature before the start of culture. Specifically, in Example 13, after the core, the alginate gel, and the gelling solution were discharged into the collection solution, the lid of the container housing the collection solution was immediately closed, and the container was incubated at 37°C for 1 hour (refer to Table 3 below). Culture of cells after incubation was performed in the same manner as in Example 11.

### [Example 14]

Example 14 will be described below. The cell capsule according to Example 14 was produced in the same manner as in Example 11 except for the incubation time before the start of culture. Specifically, in Example 14, after the core, the alginate gel, and the gelling solution were discharged into the collection solution, the lid of the container housing the collection solution was immediately closed, and the container was incubated at 4°C for 2 hours (refer to Table 3 below). Culture of cells after incubation was performed in the same manner as in Example 11.

### [Example 15]

Example 15 will be described below. The cell capsule according to Example 15 was produced in the same manner as in Example 11 except for the incubation time and the incubation temperature before the start of culture. Specifically, in Example 15, after the core, the alginate gel, and the gelling solution were discharged into the collection solution, the lid of the container housing the collection solution was immediately closed, and the container was incubated at 20°C for 2 hours (refer to Table 3 below). Culture of cells after incubation was performed in the same manner as in Example 11.

### [Example 16]

Example 16 will be described below. The cell capsule according to Example 16 was produced in the same manner as in Example 11 except for the incubation time and the incubation temperature before the start of culture. Specifically, in Example 16, after the core, the alginate gel, and the gelling solution were discharged into the collection solution, the lid of the container housing the collection solution was immediately closed, and the container was incubated at 37°C for 2 hours (refer to Table 3 below). Culture of cells after incubation was performed in the same manner as in Example 11.

### [Example 17]

Example 17 will be described below. The cell capsule according to Example 17 was produced in the same manner as in Example 11 except for the incubation time before the start of culture. Specifically, in Example 17, after the core, the alginate gel, and the gelling solution were discharged into the collection solution, the lid of the container housing the collection solution was immediately closed, and the container was incubated at 4°C for 4 hours (refer to Table 3 below). Culture of cells after incubation was performed in the same manner as in Example 11.

### [Example 18]

Example 18 will be described below. The cell capsule according to Example 18 was produced in the same manner as in Example 11 except for the incubation time and the incubation temperature before the start of culture. Specifically, in Example 18, after the core, the alginate gel, and the gelling solution were discharged into the collection solution, the lid of the container housing the collection solution was immediately closed, and the container was incubated at 20°C for 4 hours (refer to Table 3 below). Culture of cells after incubation was performed in the same manner as in Example 11.

### [Example 19]

Example 19 will be described below. The cell capsule according to Example 19 was produced in the same manner as in Example 11 except for the incubation time and the incubation temperature before the start of culture. Specifically, in Example 19, after the core, the alginate gel, and the gelling solution were discharged into the collection solution, the lid of the container housing the collection solution was immediately closed, and the container was incubated at 37°C for 4 hours (refer to Table 3 below). Culture of cells after incubation was performed in the same manner as in Example 11.

### [Example 20]

Example 20 will be described below. The cell capsule according to Example 20 was produced in the same manner as in Example 11 except for the incubation time before the start of culture. Specifically, in Example 20, after the core, the alginate gel, and the gelling solution were discharged into the collection solution, the lid of the container housing the collection solution was immediately closed, and the container was incubated at 4°C for 6 hours (refer to Table 3 below). Culture of cells after incubation was performed in the same manner as in Example 11.

### [Example 21]

Example 21 will be described below. The cell capsule according to Example 21 was produced in the same manner as in Example 11 except for the incubation time and the incubation temperature before the start of culture. Specifically, in Example 21, after the core, the alginate gel, and the gelling solution were discharged into the collection solution, the lid of the container housing the collection solution was immediately closed, and the container was incubated at 20°C for 6 hours (refer to Table 3 below). Culture of cells after incubation was performed in the same manner as in Example 11.

### [Example 22]

Example 22 will be described below. The cell capsule according to Example 22 was produced in the same manner as in Example 11 except for the incubation time and the incubation temperature before the start of culture. Specifically, in Example 22, after the core, the alginate gel, and the gelling solution were discharged into the collection solution, the lid of the container housing the collection solution was immediately closed, and the container was incubated at 37°C for 6 hours (refer to Table 3 below). Culture of cells after incubation was performed in the same manner as in Example 11.

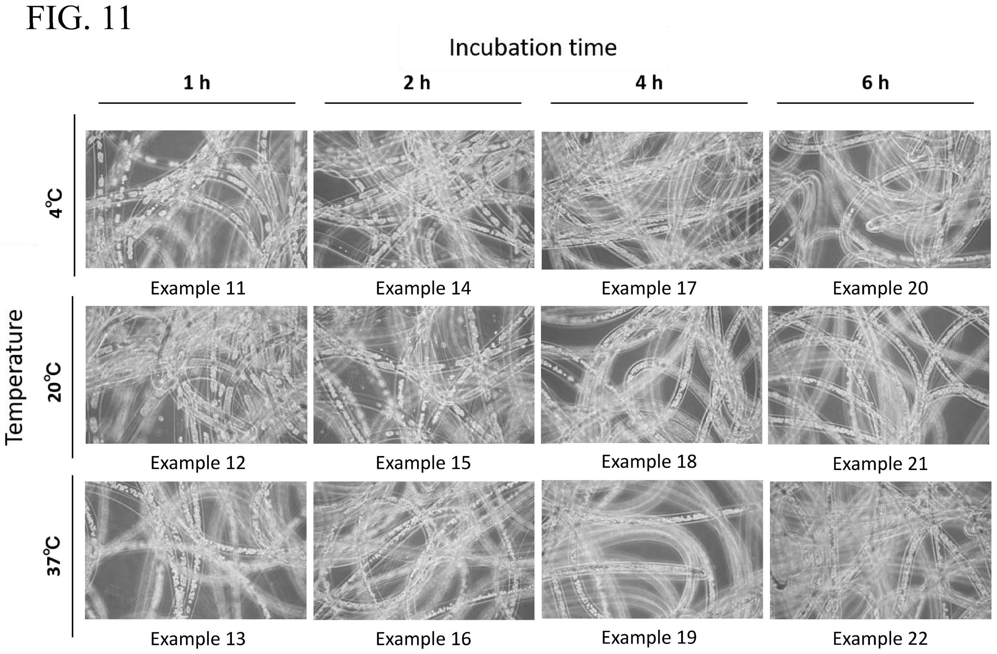

Fig. 11 is an enlarged photograph showing cell capsules on Day 2 in Examples 11 to 22. As shown in Fig. 11, it can be observed that K562 cells are housed inside the tubular alginic acid.

### [Reference Examples 11 to 22]

Reference Examples 11 to 22 will be described below. The cell capsules according to Reference Examples 11 to 22 were produced in the same manner as Examples 11 to 22, respectively, except for the composition of the collection solution. The collection solution according to Reference Examples 11 to 22 is an aqueous solution of 100 mM calcium chloride. Incubation of cell capsules in the collection solution and culture of cells after incubation were performed in the same manner as in Examples 11 to 22 (refer to Table 3 below).

**(Table 3)**

| | Cell | Composition of Collection Solution | Incubation temperature before starting the culture | Incubation time before starting the culture |
|---|---|---|---|---|
| Example 11 | K562 | Culture Medium | 4°C | 1 hour |
| Example 12 | K562 | Culture Medium | 20°C | 1 hour |
| Example 13 | K562 | Culture Medium | 37°C | 1 hour |
| Example 14 | K562 | Culture Medium | 4°C | 2 hours |
| Example 15 | K562 | Culture Medium | 20°C | 2 hours |
| Example 16 | K562 | Culture Medium | 37°C | 2 hours |
| Example 17 | K562 | Culture Medium | 4°C | 4 hours |
| Example 18 | K562 | Culture Medium | 20°C | 4 hours |
| Example 19 | K562 | Culture Medium | 37°C | 4 hours |
| Example 20 | K562 | Culture Medium | 4°C | 6 hours |
| Example 21 | K562 | Culture Medium | 20°C | 6 hours |
| Example 22 | K562 | Culture Medium | 37°C | 6 hours |
| Reference Example 11 | K562 | CaCl₂ | 4°C | 1 hour |
| Reference Example 12 | K562 | CaCl₂ | 20°C | 1 hour |
| Reference Example 13 | K562 | CaCl₂ | 37°C | 1 hour |
| Reference Example 14 | K562 | CaCl₂ | 4°C | 2 hours |
| Reference Example 15 | K562 | CaCl₂ | 20°C | 2 hours |
| Reference Example 16 | K562 | CaCl₂ | 37°C | 2 hours |
| Reference Example 17 | K562 | CaCl₂ | 4°C | 4 hours |
| Reference Example 18 | K562 | CaCl₂ | 20°C | 4 hours |
| Reference Example 19 | K562 | CaCl₂ | 37°C | 4 hours |
| Reference Example 20 | K562 | CaCl₂ | 4°C | 6 hours |
| Reference Example 21 | K562 | CaCl₂ | 20°C | 6 hours |
| Reference Example 22 | K562 | CaCl₂ | 37°C | 6 hours |

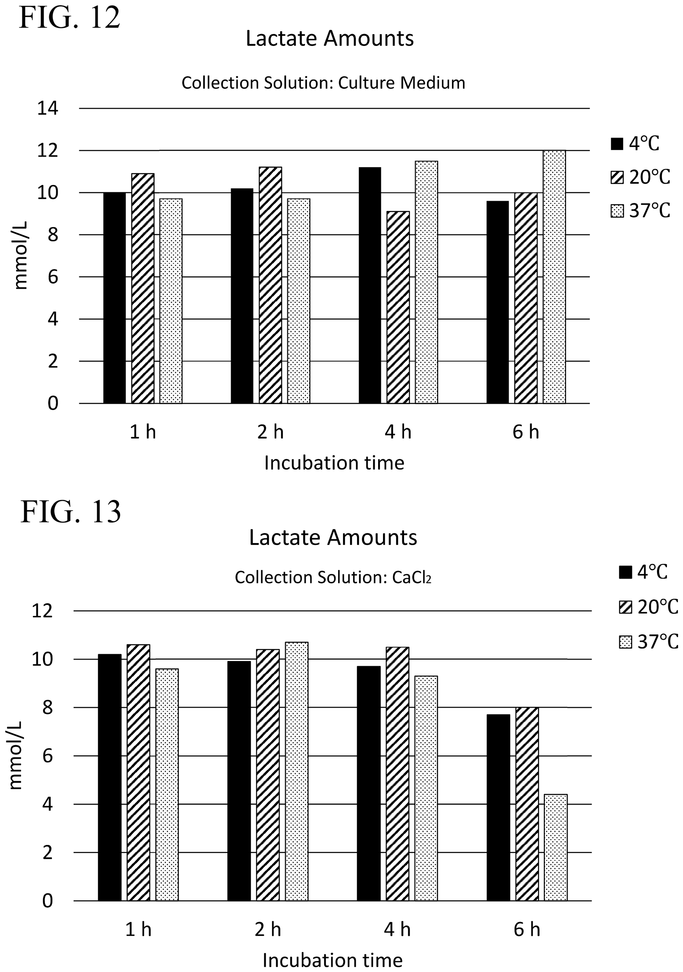

Fig. 12 is a graph showing measurement results of the lactate amount in the culture medium of K562 cells on Day 2 in Examples 11 to 22. In Fig. 12, the lactate amounts measured in Example 11, Example 12, Example 13, Example 14, Example 15, Example 16, Example 17, Example 18, Example 19, Example 20, Example 21, and Example 22 are shown in order from the left.

Fig. 13 is a graph showing the measurement results of the lactate amount in the culture medium of K562 cells on Day 2 in Reference Examples 11 to 22. In Fig. 13, the lactate amounts measured in Reference Example 11, Reference Example 12, Reference Example 13, Reference Example 14, Reference Example 15, Reference Example 16, Reference Example 17, Reference Example 18, Reference Example 19, Reference Example 20, Reference Example 21, and Reference Example 22 are shown in order from the left.

Referring to Fig. 13, it can be seen that, in the reference example, in a case where the incubation time is 6 hours, the lactate amount in the culture medium decreases at any incubation temperature. On the other hand, in Examples, even in a case where the incubation time is 6 hours, the lactate amount in the culture medium is maintained. This is considered to be because, in Examples 11 to 22, the collection solution was a culture medium having buffering capacity, and the hydrogen ion index in the collection solution was maintained relatively stably.

From the above Examples, it can be seen that the collection solution preferably contains a pH buffer in a case where it is necessary to leave the cells covered with the tubular hydrogel in the collection solution for a long time. In this case, the collection solution may be a culture medium having pH buffering capacity. In particular, it can be seen that, from the above Examples, it is preferable that such a collection solution is maintained at a relatively low temperature, for example, 40°C or lower, 30°C or lower, preferably 25°C or lower, and more preferably 20°C or lower at the time of producing the cell capsule.

Such a collection solution can be suitably utilized in producing very long cell capsules and/or large amounts of cell capsules. In the case of producing very long cell capsules and/or large amounts of cell capsules, the time for discharging the core, the hydrogel or the hydrogel precursor, the gelling solution may reach, for example, 0.25 hours or more, 0.5 hours or more, or 1 hour or more. In this case, the part of the hydrogel or the hydrogel precursor immersed in the collection solution at the start of discharge is immersed in the collection solution for a long period of time. Even in such a case, by using the collection solution containing the pH buffer, it is possible to mitigate an adverse effect on the cells in the cell capsule.

As described above, the contents of the present invention have been disclosed through the embodiments, but it should not be understood that the description and the drawings constituting a part of the disclosure limit the present invention. From this disclosure, various alternative embodiments, examples, and operational techniques will become apparent to those skilled in the art. Therefore, the technical scope of the present invention is defined only by the matters specifying the invention according to the claims appropriate from the above description.

This application claims priority based on Japanese Patent Application No. 2023-104592 filed on June 26, 2023, the entire contents of which are incorporated herein by reference.

## Claims

1. A method for producing a cell capsule, comprising
continuously or intermittently discharging a hydrogel or a hydrogel precursor covering a cell into a collection solution, wherein
the collection solution contains a pH buffer.

2. The method for producing a cell capsule according to claim 1, wherein
the pH buffer contains a Good's buffer.

3. The method for producing a cell capsule according to claim 1 or 2, comprising
maintaining the hydrogel or a hydrogel generated from the hydrogel precursor in the collection solution for 0.25 hours or more from a start of discharge of the hydrogel or the hydrogel precursor.

4. The method for producing a cell capsule according to claim 1 or 2, comprising
maintaining the hydrogel or a hydrogel generated from the hydrogel precursor in the collection solution for 1 hour or more and 24 hours or less from a start of discharge of the hydrogel or the hydrogel precursor.

5. The method for producing a cell capsule according to any one of claims 1 to 4, wherein
an average temperature of the collection solution is 0°C or more and 40°C or less.

6. The method for producing a cell capsule according to any one of claims 1 to 4, wherein
an average temperature of the collection solution is 0°C or more and 20°C or less.

7. The method for producing a cell capsule according to any one of claims 1 to 6, wherein
a pH of the collection solution is in a range of 6.5 to 8.0.

8. The method for producing a cell capsule according to any one of claims 1 to 7, wherein
the hydrogel or the hydrogel precursor covers a core containing the cell and is discharged into the collection solution together with the core.

9. The method for producing a cell capsule according to any one of claims 1 to 8, wherein
the collection solution contains a gelling agent that gels the hydrogel precursor.

10. The method for producing a cell capsule according to any one of claims 1 to 9, wherein
a gelling solution covering the hydrogel or the hydrogel precursor is discharged into the collection solution together with the hydrogel or the hydrogel precursor.

11. The method for producing a cell capsule according to claim 10, wherein
the gelling solution contains a pH buffer.

12. The method for producing a cell capsule according to claim 10 or 11, wherein
the pH buffer contains a Good's buffer.

13. The method for producing a cell capsule according to any one of claims 10 to 12, wherein
the gelling solution contains the same pH buffer as that included in the collection solution.

14. A method for producing a cell population or a cell product, comprising
culturing cells inside a hydrogel using the cell capsule produced by the method for producing a cell capsule according to any one of claims 1 to 13.

15. A cell population produced by the method for producing a cell population or a cell product according to claim 14.

16. A solution used for producing a cell capsule having a hydrogel covering a cell, comprising
a pH buffer.

17. The solution according to claim 16, wherein
the pH buffer is a Good's buffer.

18. The solution according to claim 16 or 17, containing a gelling agent that gels a hydrogel precursor.

19. The solution according to any one of claims 16 to 18, wherein
a pH of the solution is in a range of 6.5 to 8.0.
